# EUROPEAN PATENT APPLICATION

(11) **EP 4 242 230 A1**
(43) Date of publication of application: **13.09.2023**
(21) Application number: 21888618.2
(22) Date of filing: 04.11.2021
(51) Int. Cl.: C07K 16/28, C07K 19/00, C12N 5/10, A61K 39/395, A61P 17/06, G01N 33/68, G01N 33/577

(54) **ANTIBODY TARGETING INTERLEUKIN 36R, PREPARATION METHOD THEREFOR, AND APPLICATION THEREOF**

(30) Priority: 04.11.2020 CN 202011213729
(71) Applicant: Shanghai Huaota Biopharmaceutical Co., Ltd., Shanghai 201203 (CN); Huabo Biopharm (Shanghai) Co., Ltd., Shanghai 201203 (CN)
(72) Inventor: FENG, Yujie, Shanghai 201203 (CN); YU, Haijia, Shanghai 201203 (CN); WEI, Xiaoyue, Shanghai 201203 (CN); PENG, Guoyuan, Shanghai 201203 (CN); CHEN, Shi, Shanghai 201203 (CN); QU, Lili, Shanghai 201203 (CN)
(74) Representative: Weickmann & Weickmann PartmbB
(86) International application number: PCT/CN2021/128704
(87) International publication number: WO 2022/095926

(57) **Abstract**

The present application provides an antibody targeting interleukin 36R, a preparation method therefor, and an application thereof. Specifically, the present application provides an IL-36R antibody having high affinity and high bioactivity. The antibody can bind to IL-36R with high affinity, and can block the binding between an IL-36R ligand (α, β. γ) and the IL-36R and a signal path activated by the IL-36R ligand, thereby treating and/or preventing IL-36 related diseases.

## Description

### FIELD OF THE INTVENTION

The present application relates to the field of biomedicine, and in particular, relates to an antibody targeting interleukin 36R, a preparation method thereof and an application thereof.

### BACKGROUND OF THE INVENTION

IL-1 receptor (IL1R) family comprises at least 11 members, including IL1RI (IL1R1), IL1RII (IL1R2), ILlRAcP (IL1R3), ST2 (T1/IL1R4), IL18Ra (IL1Rrp/IL1R5), IL1Rrp2 (IL1RL2/IL1R6/IL-36R), IL18Rb (AcPL/IL1R7), IL1RAPL1 (TIGIRR2/IL1R8), and TIGIRR1 (IL1R9). IL-36R may recognize three different ligand-activating cytokines, IL36α, IL36β and IL36γ (also known as IL-1F6, IL-1F8 and IL-1F9), to trigger the expression of inflammatory cytokines. Also, there are two natural antagonists IL-36Ra (IL-1F5) and IL-38, which inhibit the expression of inflammatory cytokines after binding to IL-36R.

IL-36R is expressed on lung epithelial cells, cerebrovascular cells, kidneys, testes, monocytes, skinderived keratinocytes, fibroblasts, and endothelial cells. IL-36R consists of an extracytomembrane ligand-binding domain, a transmembrane helix, and an intracellular signal transduction Toll/IL-1 receptor domain (TIR domain). IL-36α, IL-36β, and IL-36γ first bind to IL-36R to form a signal transduction complex with IL-1RAcP, and then recruit a myeloid differentiation factor 88 (MyD88) to activate mitogen-activated protein kinase (MAPK) and nuclear factor kappa B (NF-κB) pathways mediated by c-Jun N-terminal kinases (JNKs) and extracellular regulated protein kinases (ERK1/2), producing a large number of inflammatory mediators to in turn mediate inflammatory responses, thereby playing an important role in adaptive immunity. The binding of IL-36Ra to IL-36R does not lead to the recruitment of IL-1RAcP, and the pro-inflammatory cascade reaction is not initiated, which achieves the anti-inflammatory properties of IL-36Ra. In a normal human body, the signal activation mediated by IL-36α, IL-36β, or IL-36γ and the signal inhibition mediated by IL-36Ra are in an equilibrium state, and when IL-36Ra undergoes gene mutation, its inhibitory function would be inactivated to break the equilibrium state, causing inflammatory diseases.

IL-36 cytokines are involved in some serious psoriasis, including generalized pustular psoriasis (GPP) and palmoplantar pustulosis (PPP). GPP is a severe systemic-type pustular psoriasis that is fatal. PPP is chronic pustular psoriasis affecting the palms and soles. Current therapies for GPP and PPP include oral retinoids and topical steroids, but with poor efficacy and serious side effects.

Therefore, there is still a need for an anti-IL-36R antibody that may bind to IL-36R at high affinity and block the binding of IL-36α, IL-36β, or IL-36γ to IL-36R for treatment of IL-36-related diseases such as GPP and PPP.

### SUMMARY OF THE INVENTION

An object of the present application is to provide an antibody targeting interleukin 36R, a preparation method thereof and an application thereof.

In particular, the object of the present application is to provide a high-affinity and high-biological-activity IL-36R antibody capable of binding to IL-36R at high affinity and blocking the binding of IL-36α, IL-36β, or IL-36γ to IL-36R, and an application thereof.

Another object of the present application is to provide an interleukin-36R binding molecule and uses thereof, in particular uses in the treatment and/or prevention, or diagnosis of IL-36-related diseases such as psoriasis.

The first aspect of the present application provides a heavy-chain variable region of an antibody. The heavy-chain variable region comprises the following three complementary determining regions (CDRs):
CDR1 as set forth in SEQ ID NO: 3,
CDR2 as set forth in SEQ ID NO: 4, and
CDR3 as set forth in SEQ ID NO: 5.

In some embodiments, any of the above amino acid sequences further comprises a derivative sequence that has been optionally added, deleted, modified and/or substituted with at least one (e.g., 1-3, possibly 1-2, or possibly 1) amino acid and may retain the IL-36R binding affinity.

In some embodiments, the heavy-chain variable region further comprises a human-derived FR or a murine FR.

In some embodiments, the heavy-chain variable region has an amino acid sequence as set forth in SEQ ID NO: 1.

In some embodiments, the heavy-chain variable region has an amino acid sequence as set forth in SEQ ID NO: 9.

The second aspect of the present application provides an antibody heavy chain, comprising a heavy-chain variable region as described in the first aspect of the present application.

In some embodiments, the antibody heavy chain further comprises a heavy-chain constant region.

In some embodiments, the heavy-chain constant region is human-derived, murine, or rabbit-derived. The third aspect of the present application provides a light-chain variable region of an antibody. The light-chain variable region comprises the following three complementary determining regions CDRs:
CDR1' as set forth in SEQ ID NO: 6,
CDR2' as set forth in SEQ ID NO: 7, and
CDR3' as set forth in SEQ ID NO: 8.

In some embodiments, any of the above amino acid sequences further comprises a derivative sequence that has been optionally added, deleted, modified and/or substituted with at least one (e.g., 1-3, possibly 1-2, or possibly 1) amino acid and may retain the IL-36R binding affinity.

In some embodiments, the light-chain variable region further comprises a human-derived FR or a murine FR.

In some embodiments, the light-chain variable region has an amino acid sequence as set forth in SEQ ID NO: 2.

In some embodiments, the light-chain variable region has an amino acid sequence as set forth in SEQ ID NO: 10, 11, or 12.

The fourth aspect of the present application provides an antibody light chain, comprising a light-chain variable region as described in the third aspect of the present application.

In some embodiments, the antibody light chain further comprises a light-chain constant region.

In some embodiments, the light-chain constant region is human-derived, murine, or rabbit-derived. The fifth aspect of the present application provides an antibody, comprising:
(1) the heavy-chain variable region as described in the first aspect of the present application; and/or
(2) the light-chain variable region as described in the third aspect of the present application;
or, the antibody has: the heavy chain as described in the second aspect of the present application; and/or the light chain as described in the fourth aspect of the present application.

In some embodiments, an affinity constant KD (M) for binding the antibody to a human IL-36R protein (for example, which may be a wild type) is (0.5-10) × 10⁻¹¹, which may be (1-6) × 10⁻¹¹, and may be (1-2) × 10⁻¹¹.

In some embodiments, the affinity constant KD(M) for binding the antibody to human IL-1 Rrp2 is (0.5-10) × 10⁻¹¹, which may be (1-6) × 10⁻¹¹, and may be (1-2) × 10⁻¹¹.

In some embodiments, the antibody is capable of blocking the binding of IL-36α, IL-36β, or IL-36γ to IL-36R.

In some embodiments, the antibody is capable of blocking cytokine secretion mediated by IL-36α, IL-36β, or IL-36γ, and the cytokine comprises: IL-6, IL-8, and GM-CSF.

In some embodiments, the antibody is selected from: an animal-derived antibody, a chimeric antibody, a humanized antibody, or a combination thereof.

In some embodiments, the antibody is a double-chain antibody or a single-chain antibody.

In some embodiments, the antibody is a monoclonal antibody.

In some embodiments, the antibody is a partially or fully humanized monoclonal antibody.

In some embodiments, the heavy-chain variable region of the antibody has a sequence as set forth in SEQ ID NO: 1 or 9; and/or
the light-chain variable region of the antibody has a sequence as set forth in SEQ ID NO: 2, 10, 11 or 12.

In some embodiments, the heavy-chain variable region of the antibody has a sequence as set forth in SEQ ID NO: 1; and the light-chain variable region of the antibody has a sequence as set forth in SEQ ID NO: 2.

In some embodiments, the heavy-chain variable region of the antibody has a sequence as set forth in SEQ ID NO: 9; and the light-chain variable region of the antibody has a sequence as set forth in SEQ ID NO: 10, 11 or 12.

In some embodiments, the antibody is in the form of a drug conjugate.

The sixth aspect of the present application provides a recombinant protein, comprising:
(i) the heavy-chain variable region as described in the first aspect of the present application, the heavy chain as described in the second aspect of the present application, the light-chain variable region as described in the third aspect of the present application, the light chain as described in the fourth aspect of the present application, or the antibody as described in the fifth aspect of the present application; and
(ii) an optional tag sequence assisting expression and/or purification.

In some embodiments, the tag sequence comprises a 6His tag.

In some embodiments, the recombinant protein (or polypeptide) comprises a fusion protein.

In some embodiments, the recombinant protein is a monomer, a dimer, or a multimer.

The seventh aspect of the present application provides a CAR construct, wherein an scFV segment of an antigen-binding region of a monoclonal antibody of the CAR construct is a binding region specifically binding to IL-36R, and the scFv has the heavy-chain variable region as described in the first aspect of the present application and the light-chain variable region as described in the third aspect of the present application.

The eighth aspect of the present application provides a recombinant immune cell expressing the exogenous CAR construct as described in the seventh aspect of the present application.

In some embodiments, the immune cell is selected from the group consisting of: NK cells and T cells. In some embodiments, the immune cells are derived from human or non-human mammals (such as mice).

The ninth aspect of the present application provides an antibody-drug conjugate, comprising:
(a) an antibody moiety selected from the group consisting of: the heavy-chain variable region as described in the first aspect of the present application, the heavy chain as described in the second aspect of the present application, the light-chain variable region as described in the third aspect of the present application, the light chain as described in the fourth aspect of the present application, or the antibody as described in the fifth aspect of the present application, or a combination thereof; and
(b) a conjugated moiety conjugated to the antibody moiety, the conjugated moiety being selected from the group consisting of: detectable markers, drugs, toxins, cytokines, radionuclides, enzymes, or combinations thereof.

In some embodiments, the antibody moiety is conjugated to the conjugated moiety by a chemical bond or linker.

The tenth aspect of the present application provides a use of an active ingredient selected from the group consisting of: the heavy-chain variable region as described in the first aspect of the present application, the heavy chain as described in the second aspect of the present application, the light-chain variable region as described in the third aspect of the present application, the light chain as described in the fourth aspect of the present application, or the antibody as described in the fifth aspect of the present application, the recombinant protein as described in the sixth aspect of the present application, the immune cell as described in the eighth aspect of the present application, the antibody-drug conjugate as described in the ninth aspect of the present application, or a combination thereof. The active ingredient is used for (a) preparation of a detection reagent or kit; and/or (b) preparation of a drug for prevention and/or treatment of IL-36-related diseases.

In some embodiments, the IL-36-related diseases are IL-36-mediated inflammatory diseases.

In some embodiments, the IL-36-related diseases are those caused by overstimulation or mutation of IL-36 cytokines.

In some embodiments, the IL-36-related diseases are selected from the group consisting of: inflammations, autoimmune diseases, or combinations thereof. For example, the IL-36-related diseases may be autoimmune diseases.

In some embodiments, the IL-36-related diseases are selected from the group consisting of: psoriasis, scleroderma, chronic kidney disease, inflammatory bowel disease, psoriatic arthritis, ankylosing spondylitis, multiple sclerosis, inflammatory arthritis, asthma, allergy, or a combination thereof. For example, the IL-36-related disease may be psoriasis.

In some embodiments, the psoriasis comprises psoriasis vulgaris, erythrodermic psoriasis, arthropathy psoriasis, generalized pustular psoriasis (GPP), palmoplantar pustulosis (PPP).

In some embodiments, the drug is used to block the binding of IL-36α, IL-36β, or IL-36γ to IL-36R. In some embodiments, the drug is used to block cytokine secretion medicated by IL-36α, IL-36β, or IL-36γ.

In some embodiments, the antibody is in the form of a drug conjugate (ADC).

In some embodiments, the detection reagent or kit is used to diagnose IL-36-related diseases.

In some embodiments, the detection reagent or kit is used to detect an IL-36R protein in a sample. In some embodiments, the detection reagent is a detection tablet.

The eleventh aspect of the present application provides a pharmaceutical composition, comprising:
(i) an active ingredient selected from the group consisting of: the heavy-chain variable region as described in the first aspect of the present application, the heavy chain as described in the second aspect of the present application, the light-chain variable region as described in the third aspect of the present application, the light chain as described in the fourth aspect of the present application, or the antibody as described in the fifth aspect of the present application, the recombinant protein as described in the sixth aspect of the present application, the immune cell as described in the eighth aspect of the present application, the antibody-drug conjugate as described in the ninth aspect of the present application, or a combination thereof; and
(ii) a pharmaceutically acceptable carrier.

In some embodiments, the pharmaceutical composition is a liquid preparation.

In some embodiments, the pharmaceutical composition is an injection.

The twelfth aspect of the present application provides a polynucleotide encoding a polypeptide selected from the group consisting of:
(1) the heavy-chain variable region as described in the first aspect of the present application, the heavy chain as described in the second aspect of the present application, the light-chain variable region as described in the third aspect of the present application, the light chain as described in the fourth aspect of the present application, or the antibody as described in the fifth aspect of the present application; or
(2) the recombinant protein as described in the sixth aspect of the present application;
(3) the CAR construct as described in the seventh aspect of the present application.

The thirteenth aspect of the present application provides a vector comprising the polynucleotide as described in the twelfth aspect of the present application.

In some embodiments, the vector comprises: bacterial plasmids, bacteriophages, yeast plasmids, plant cell viruses, mammalian cellular viruses such as adenoviruses, retroviruses, or other vectors.

The fourteenth aspect of the present invention provides a genetically engineered host cell, comprising the vector as described in the thirteenth aspect of the present application or a genome integrated with the polynucleotide as described in the twelfth aspect of the present application.

The fifteenth aspect of the present application provides a method for *in vitro* detection (including diagnostic or non-diagnostic detection) of an IL-36R protein in a sample, comprising the steps of:
(1) contacting, *in vitro,* the sample with the antibody as described in the fifth aspect of the present application or the antibody-drug conjugate as described in the ninth aspect of the present application; and
(2) detecting whether an antigen-antibody complex is formed, wherein the formation of the complex indicates the presence of the IL-36R protein in the sample.

In some embodiments, the method is for non-diagnostic treatment.

The sixteenth aspect of the present application provides a detection plate, comprising: a substrate (support plate) and a detection strip, wherein the detection strip comprises the antibody as described in the fifth aspect of the present application or the antibody-drug conjugate as described in the ninth aspect of the present application.

The seventeenth aspect of the present application provides a kit, comprising:
(1) a first container comprising the antibody as described in the fifth aspect of the present application; and/or
(2) a second container comprising a secondary antibody against the antibody as described in the fifth aspect of the present application;
or, the kit comprises the detection plate as described in the sixteenth aspect of the present application. The eighteenth aspect of the present application provides a preparation method for a recombinant polypeptide, comprising the steps of:
(a) culturing the host cell as described in the fourteenth aspect of the present application under conditions suitable for expression; and
(b) isolating a recombinant polypeptide from a culture, the recombinant polypeptide being the antibody as described in the fifth aspect of the present application or the recombinant protein as described in the sixth aspect of the present application.

The nineteenth aspect of the present application provides a method for preventing/or treating IL-36-related diseases, comprising: administering the antibody as described in the fifth aspect of the present application, an antibody-drug conjugate of the antibody, or a CAR-T cell expressing the antibody, or a combination thereof, to a subject in need thereof.

In some embodiments, the method further comprises: administering other drugs or therapies to the subject in need thereof for combination treatment.

It should be understood that, within the scope of the present application, the above technical features of the present application and the technical features specifically described hereinafter (such as in embodiments) may be combined with each other to accordingly constitute a new technical solution. Due to space limitation, the details will not be repeated one by one here.

### BRIEF DESCRIPTION OF THE DRAWINGS

The specific features of the present invention involved in the present application are listed in the appended claims. The characteristics and advantages of the present invention involved in the present application may be better understood by referring to the exemplary embodiments and the accompanying drawings described in detail below. A brief description of the drawings is as follows:
FIG. 1 shows the results of cytokine IL-6 release in a bioactivity detection for candidate humanized antibodies;
FIG. 2 shows the results of cytokine IL-8 release in a bioactivity detection for candidate humanized antibodies;
FIG. 3 shows the results of cytokine GM-CSF release in a bioactivity detection for candidate humanized antibodies;
FIG. 4 shows the results of NF-κB phosphorylation in a bioactivity detection for candidate humanized antibodies;
FIG. 5 shows the cell binding activity of candidate antibodies to human IL-36R;
FIG. 6 shows the cell binding activity of candidate antibodies to monkey IL-36R;
FIG. 7 shows the functional inhibition effect of candidate antibodies against huIL-36 stimulating factors in NCI/ADR-RES cells;
FIG. 8 shows the functional inhibition effect of candidate antibodies against huIL-36 stimulating factors in HIF cells;
FIG. 9 shows the functional inhibition effect of candidate antibodies against huIL-36 stimulating factors in HIF cells;
FIG. 10 shows the functional inhibition effect of candidate antibodies against huIL-36 stimulating factors in HIF cells;
FIG. 11 shows the effect of candidate antibodies on the clinical scoring (PASI) of cynomolgus monkeys with imiquimod-induced psoriasis; and
FIG. 12 shows the effect of candidate antibodies on the HE-staining pathological scoring of skin tissues of cynomolgus monkey with imiquimod-induced psoriasis.

### DETAILED DESCRIPTION OF THE PREFERRED EMBODIMENTS

The embodiments of the invention of the present application will be illustrated by specific examples below. Those familiar with this technology may easily understand other advantages and effects of the invention of the present application from the content disclosed in the specification.

### Terms and Definitions

Based on extensive and in-depth researches, the applicants have unexpectedly obtained an IL-36R antibody with high affinity and high bioactivity. Experiments have shown that the IL-36R antibody of the present application may bind to IL-36R at high affinity, and block the binding of IL-36R ligands (α, β, γ) to IL-36R to block the signaling pathway activated by the IL-36R ligands, thereby treating and/or preventing IL-36-related diseases. The present invention is implemented on this basis.

### Terms

As used herein, the terms "administration" and "treatment" refer to the application of exogenous medicaments, therapeutic agents, diagnostic agents, or compositions to animals, humans, subjects, cells, tissues, organs, or biological fluids. "Administration" and "treatment" may refer to treatment, pharmacokinetics, diagnosis, studies, and experimental methods. The treatment of cells comprises contact between reagents and cells, contact between reagents and fluids, and contact between fluids and cells. "Administration" and "treatment" also mean *in vitro* and *ex vivo* treatment by means of reagents, diagnostics, and binding compositions, or by another cell. When applied to humans, animals, or research subjects, "treatment" refers to therapeutic treatment, prevention or preventive measures, study, and diagnosis. It encompasses the contact between IL-36R antibodies and humans or animals, subjects, cells, tissues, physiological compartments, or physiological fluids.

As used herein, the term "therapy" refers to the administration of an internally or externally applied therapeutic agent, which comprises any of the IL-36R antibodies and compositions thereof in the present application, to a patient who has one or more disease symptoms, and furthermore, the therapeutic agent is known to show a therapeutic effect against these symptoms. The therapeutic agent is usually administered to the patient at an amount (therapeutically effective amount) for effectively alleviating one or more disease symptoms.

As used herein, the term "optional" or "optionally" means that the event or situation described subsequently may occur but does not have to occur. For example, "optionally comprising 1 to 3 antibody heavy-chain variable regions" refers to that a particular sequence may have but not necessarily have 1, 2 or 3 antibody heavy-chain variable regions.

### Antibody

As used herein, the term "antibody" refers to immunoglobulin, which is a tetrapeptide chain structure connected by interchain disulfide bonds between two identical heavy chains and two identical light chains. Different immunoglobulin heavy chain constant regions exhibit different amino acid compositions and rank orders, thereby presenting different antigenicity. Accordingly, immunoglobulins may be divided into five categories, or called immunoglobulin isotypes, namely IgM, IgD, IgG; IgA and IgE, which correspond to heavy-chain constant regions, called α, δ, ε, γ and µ, of different types of immunoglobulins. IgG represents one of the most important classes of immunoglobulins, and due to differences in its chemical structure and biological function, it can be divided into four sub-categories: IgG1, IgG2, IgG3, and IgG4. Light chains are divided into κ or λ chains considering of different constant regions. The subunit structures and three-dimensional configurations of immunoglobulins in different categories are well known to those in the art.

Near the N-terminal of the antibody heavy chains and light chains, the sequence of about 110 amino acids varies largely, known as the variable region (V region); and the rest of the amino acid sequence near the C-terminal is relative stable, known as the constant region (C region). The variable region comprises three hypervariable regions (HVR) and four FRs with a relatively conserved sequence. The amino acid sequence of the four FRs are relatively conserved and are not directly involved in a binding reaction. The three hypervariable regions determine the specificity of the antibody, also known as complementary determining regions (CDRs). Each light-chain variable region (LCVR) and each heavy-chain variable region (HCVR) are composed of three CDRs and four FRs, with a sequential rank order from the amino terminal to the carboxyl terminal being: FR1, CDR1, FR2, CDR2, FR3, CDR3, and FR4. The three CDRs of the light chain, namely the light-chain hypervariable regions (LCDRs), refer to LCDR1, LCDR2 and LCDR3; and the three CDRs of the heavy chain, namely the heavy-chain highly variable regions (HCDRs), refer to HCDR1, HCDR2 and HCDR3. The numbers and locations of CDR amino acid residues in the LCVRs and HCVRs of the antibody or antigen-binding fragment of the present invention correspond with known Kabat numbering scheme (LCDR1 - 3, HCDE2-3), or correspond with kabat and chothia numbering schemes (HCDR1). The four FRs in the natural heavy-chain and light-chain variable regions are roughly in a β-folded configuration linked by three CDRs that form a linking ring, and in some cases, the four FRs may form a partially β-folded structure. The CDRs in each chain are closely brought together by the FRs, and form, together with the CDRs from another chain, an antigen-binding site of the antibody. It is possible to determine which amino acids make up the FRs or CDRs by comparing the amino acid sequences of antibodies of the same type. The constant regions are not directly involved in the binding between an antibody and an antigen, but they exhibit different effector functions, for example, participating in the antibodydependent cytotoxicity of the antibody.

As used herein, the term "antigen-binding fragment" refers to a Fab fragment, a Fab' fragment, a F(ab')2 fragment, or a single Fv fragment, having an antigen-binding activity. The Fv antibody contains a heavy chain variable region and a light chain variable region, but does not have a constant region, and has a minimum antibody fragment with all antigen binding sites. Generally, the Fv antibody further contains a polypeptide linker between the VH and VL domains, and can form a structure required for antigen binding.

As used herein, the term "antigenic determinant" refers to three-dimensional spatial sites distributed discontinuously on an antigen and identified by the antibody or antigen-binding fragment of the present application.

The present application includes not only intact antibodies, but also fragments of immunocompetent antibodies or fusion proteins formed by antibodies with other sequences. Therefore, the present application further comprises fragments, derivatives, and analogues of the antibody.

In the present application, the antibody comprises murine, chimeric, humanized or fully human antibodies prepared with techniques familiar to those skilled in the art. Recombinant antibodies, for example, chimeric and humanized monoclonal antibodies (including human and non-human moieties), can be prepared using the recombinant DNA techniques well known in the art.

As used herein, the term "monoclonal antibody" refers to an antibody secreted by a clone derived from a single cell source. The monoclonal antibody is highly specific for a single epitope. The cells may be eukaryotic, prokaryotic, or phage clonal cell lines.

As used herein, the term "chimeric antibody" is an antibody molecule expressed by splicing the V region gene of a murine antibody and the C region gene of a human antibody into a chimeric gene, and then inserting the chimeric gene into a vector to transfect a host cell. It not only retains the high specificity and affinity of parental mouse antibody, but also enables its human-derived Fc segment to effectively mediate the biological effector functions.

As used herein, the term "humanized antibody" is a variable region modification form of the murine antibody of the present application. It has CDRs derived from (or substantially derived from) a non-human antibody (e.g., which may be a mouse monoclonal antibody), and FRs and constant regions substantially derived from the human antibody sequence. That is, the CDR sequences of the murine antibody are grafted onto different types of human germline antibody framework sequences. Since the CDR sequences are responsible for most of the antibody-antigen interactions, recombinant antibodies that mimic the properties of specific naturally-occurring antibodies can be expressed by constructing expression vectors.

In the present application, the antibody may be monospecific, bispecific, trispecific, or more multispecific.

In the present application, the antibody of the present application further comprises conserved variants thereof, which refer to polypeptides formed by substituting up to 10, possibly up to 8, possibly up to 5, and possibly up to 3 amino acids with amino acids having similar or resembling properties, as compared with the amino acid sequence of the antibody of the present application. These conserved variant peptides are best produced by amino acid substitution according to Table A.

**Table A**

| Initial residue | Representative substituent | Substituted with, for example |
|---|---|---|
| Ala (A) | Vai; Leu; He | Vai |
| Arg (R) | Lys; Gin; Asn | Lys |
| Asn (N) | Gin; His; Lys; Arg | Gin |
| Asp (D) | Glu | Glu |
| Cys (C) | Ser | Ser |
| Gin (Q) | Asn | Asn |
| Glu (E) | Asp | Asp |
| Gly (G) | Pro; Ala | Ala |
| His (H) | Asn; Gin; Lys; Arg | Arg |
| He (1) | Leu; Vai; Met; Ala; Phe | Leu |
| Leu (L) | He; Vai; Met; Ala; Phe | Ile |
| Lys (K) | Arg; Gin; Asn | Arg |
| Met (M) | Leu; Phe; He | Leu |
| Phe (F) | Leu; Vai; lie; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Vai (V) | He; Leu; Met; Phe; Ala | Leu |

### Anti-IL-36R Antibody

As used herein, the term "IL-36R" generally refers to natural or recombinant human IL-36R, as well as non-human homologues of human IL-36R. Unless otherwise indicated, the molar concentration of IL-36R is calculated using the molecular weight of the homodimer of IL-36R.

The present application provides a high-specificity and high-affinity antibody targeting IL-36R. The antibody comprises a heavy chain and a light chain; the heavy chain comprises a heavy-chain variable region (VH) amino acid sequence, and the light chain comprising a light-chain variable region (VL) amino acid sequence.

For example, the CDR of the heavy-chain variable region (VH) is selected from the group consisting of:
CDR1 as set forth in SEQ ID NO: 3,
CDR2 as set forth in SEQ ID NO: 4, and
CDR3 as set forth in SEQ ID NO: 5; and/or
the CDR of the light-chain variable region (VL) is selected from the group consisting of:
   CDR1' as set forth in SEQ ID NO: 6,
   CDR2' as set forth in SEQ ID NO: 7, and
   CDR3' as set forth in SEQ ID NO: 8.

Here, any of the above amino acid sequences further comprises a derivative sequence which has been added, deleted, modified and/or substituted with at least one (such as 1-5, 1-3, possibly 1-2, or possibly 1) amino acid and has IL-36R binding affinity.

In some embodiments, the sequence homology achieved by the addition, deletion, modification, and/or substitution of at least one amino acid sequence may be at least 80%, may be at least 85%, may be at least 90%, or may be at least 95% of the amino acid sequence.

The antibody of the present application may be a double-chain or single-chain antibody, and may be selected from animal-derived antibodies, chimeric antibodies, and humanized antibodies. It may be a humanized antibody or a human-animal chimeric antibody, or may be a fully humanized antibody. The antibody derivatives described in the present application may be single-chain antibodies, and/or antibody fragments (such as: Fab, Fab', (Fab')₂ or other antibody derivatives known in the art), and IgA, IgD, IgE, IgG and IgM antibodies or any one or more of other subtypes of antibodies.

Here, the animal may be a mammal, such as a mouse.

The antibody of the present application may be a murine antibody, chimeric antibody, humanized antibody, or CDR-grafted and/or -modified antibody targeting human IL-36R.

In some cases, any one or more sequences of the above SEQ ID NO: 3, 4 and 5, or their sequences that have been added, deleted, modified and/or substituted with at least one amino acid and have IL-36R binding affinity, are located in the CDR of the heavy-chain variable region (VH).

In some cases, any one or more sequences of the above SEQ ID NO: 6, 7 and 8, or their sequences that have been added, deleted, modified and/or substituted with at least one amino acid and have IL-36R binding affinity, are located in the CDR of the light-chain variable region (VL).

In some cases, VH CDR1, CDR2, and CDR3 are each independently selected from any one or more sequences of SEQ ID NO: 3, 4, and 5, or their sequences that have been added, deleted, modified and/or substituted with at least one amino acid and have IL-36R binding affinity; and VL CDR1, CDR2, and CDR3 are each independently selected from any one or more sequences of SEQ ID NO: 6, 7, and 8, or their sequences that have been added, deleted, modified and/or substituted with at least one amino acid and have IL-36R binding affinity.

In the above description of the present application, the number of the amino acids added, deleted, modified and/or substituted, for example, may not exceed 40%, may not exceed 35%, may be 1-33%, may be 5-30%, may be 10-25%, or may be 15-20%, of the total number of amino acids in an initial amino acid sequence.

In the present application, the number of the amino acids added, deleted, modified and/or substituted is usually 1, 2, 3, 4 or 5, which may be 1-3, may be 1-2, or may be 1.

### Preparation of Antibodies

Any method suitable for producing a monoclonal antibody can be used to produce the IL-36R antibody of the present application. For example, animals can be immunized with linked or naturally occurring IL-36R homodimers or fragments thereof. Suitable immunization methods may be used, including adjuvants, immunostimulants, and repeated booster immunizations, and one or more routes may be used.

Any suitable form of IL-36R may be used as an immunogen (antigen) to produce a non-human antibody specific to IL-36R and to screen the biological activites of the antibody. An eliciting immunogen may be a full-length mature human IL-36R, comprising natural homodimers, or peptides containing single/multiple epitopes. The immunogen may be used alone, or in combination with one or more immunogenicity enhancers known in the art. The immunogen may be purified from a natural source, or produced in a genetically modified cell. An DNA encoding the immunogen may be genomic or non-genomic (for example, cDNA) in origin. A suitable genetic vector may be used to express the DNA encoding the immunogen, and the vector comprises, but is not limited to, an adenovirus vector, an adeno-associated virus vector, a baculovirus vector, a material, and a non-viral vector.

An exemplary method for producing the anti-human IL-36R antibody of the present application is described in Example 1.

A humanized antibody may be selected from any class of immunoglobulin, including IgM, IgD, IgG, IgA, and IgE. In the present application, the antibody is an IgG antibody, and an IgG1 subtype is used. With the biological assays described in the examples below, it is easy to optimize an essential constant domain sequence by screening antibodies, so as to produce the desired bioactivity.

Similarly, any type of light chain can be used in the compounds and methods herein. Specifically, κ and λ chains or their variants may be used in the compounds and methods of the present application. An exemplary method for humanizing the anti-human IL-36R antibody of the present application is described in Example 2.

The sequence of the DNA molecule of the antibody or the fragment thereof in the present application can be obtained by conventional techniques, such as methods using PCR amplification or genomic library screening and the like. In addition, the coding sequences of the light and heavy chains may also be fused together to form a single-chain antibody.

Once relevant sequences are obtained, they may be obtained on a large scale by recombination. This is usually achieved by cloning them into vectors, then transferring them into cells, and then isolating the relevant sequences from the proliferated host cell by means of a conventional method.

In addition, the relevant sequences may also be synthesized by using an artificial synthesis method, in particular when a fragment is short. A fragment with a very long sequence may be usually obtained by first synthesizing multiple small fragments, and then linking these small fragments. Then, this DNA sequence may be introduced into various existing DNA molecules (or such as vectors) and cells known in the art.

The present application further relates to vectors comprising the aforementioned appropriate DNA sequences and appropriate promoters or control sequences. These vectors may be used to transform appropriate host cells to enable them to express proteins.

The host cells may be prokaryotic cells, such as bacterial cells; or lower eukaryotic cells, such as yeast cells; or higher eukaryotic cells, such as mammalian cells. The animal cells may comprise (but are not limited to): CHO-S, CHO-Kl, and HEK-293 cells.

The step of transforming the host cells with recombinant DNAs in the present application may be performed using techniques well known in the art. A resulting transformant may be cultured by a conventional method, and it expresses the polypeptide encoded by the gene of the present application. According to the host cells used, they are cultured in a conventional medium under suitable conditions. Usually, the host cells are cultured and transformed under conditions suitable for the expression of the antibody of the present application. Then, the antibody of the present application is purified and obtained using conventional immunoglobulin purification steps, such as protein A-Sepharose, hydroxyapatite chromatography, gel electrophoresis, dialysis, ion exchange chromatography, hydrophobic chromatography, molecular sieve chromatography, or affinity chromatography, and other conventional separation and purification means well known to those skilled in the art.

The resulting monoclonal antibody may be identified by a conventional means. For instance, the binding specificity of the monoclonal antibody may be determined by immunoprecipitation or *in vitro* binding assays, such as radioimmunoassay (RIA) or enzyme-linked immunosorbent assay (ELISA).

### Antibody-drug Conjugate (ADC)

The present application further provides an antibody-drug conjugate (ADC) based on the antibody of the present application.

Typically, the antibody-drug conjugate comprises the antibody, and an effector molecule. The antibody is conjugated or may be chemically conjugated to the effector molecule. Here, the effector molecule may be a drug having a therapeutic activity. Furthermore, the effector molecule may be one or more of a toxic protein, a chemotherapy drug, a small molecule drug, or a radionuclide.

The antibody of the present application and the effector molecule may be conjugated with each other by a conjugating agent. Examples of the conjugating agent may be any one or more of a non-selective conjugating agent, a conjugating agent using a carboxyl group, a peptide chain, a conjugating agent using a disulfide bond. The non-selective conjugating agent refers to a compound such as glutaraldehyde, which allows the effector molecule and the antibody to be linked via a covalent bond. The conjugating agent using the carboxyl group may be any or more of a cis- aconitic anhydridebased conjugating agent (e.g., cis-aconitic anhydride), an acylhydrazone-based conjugating agent (with acylhydrazone as a conjugating site).

Some residues (such as Cys or Lys) on the antibody are used to link a variety of functional groups, involving an imaging reagent (e.g., a chromophore and a fluorophore), a diagnostic reagent (e.g., an MRI contrast agent and a radioisotope), a stabilizer (e.g., a glycol polymer), and a therapeutic agent. The antibody may be conjugated to a functional agent to form an antibody-functional agent conjugate. The functional agent (e.g., a drug, a detection reagent, or a stabilizer) is conjugated (covalently linked) to the antibody. The functional agent may be attached to the antibody directly or indirectly via a linker. The antibody may conjugate a drug to form an antibody-drug conjugate (ADC). Typically, the ADC comprises a linker between the drug and the antibody. The linker may be degradable or non-degradable. The degradable linker typically degrades easily in an intracellular environment, for example, at a site of interest, to release the drug from the antibody. Suitable degradable linkers include, for example, enzymatically degradable linkers, including peptidyl-containing linkers degradable by intracellular proteases (e.g., lysosomal proteases or endosomal proteases); or saccharide linkers, for example, glucuronide-containing linkers degradable by glucuronidases. The peptidyl-containing linkers may include, for example, dipeptides, such as valine-citrulline, phenylalanine-lysine, or valine-alanine. Other suitable degradable linkers include, for example, pH-sensitive linkers (e.g., linkers hydrolyzed at pH less than 5.5, for example, hydrazone linkers) and linkers (e.g., disulfide bond linkers) degrading under reducing conditions. Nondegradable linkers typically release drugs under conditions where antibodies are hydrolyzed by proteases.

Before linking the antibody, the linker has an active reactive group capable of reacting with some amino acid residues, and the linkage is achieved by the active reactive group. Sulfhydryl-specific active reactive groups may include: for example, maleimide-based compounds, halogenated amides (e.g., iodo-, bromo- or chlorinated amides); halogenated esters (e.g. iodo-, bromo- or chlorinated esters); halogenated methylketones (e.g. iodo-, bromo- or chlorinated methylketones), benzyl halides (e.g. iodides, bromides or chlorides); vinyl sulfone, pyridyl disulfide; mercury derivatives, for example, 3,6-bis-(mercury methyl)dioxane, with acetates, chloride ions or nitrates as p-ions; and polymethylene dimethyl sulfide thiosulfonates. The linkers may include, for example, maleimide attached to the antibody by thiosuccinimide.

The drug may be any cytotoxic, cell-growth inhibitory, or immunosuppressive drug. In an embodiment, the linker links the antibody and the drug, and the drug has a functional group that can be bonded with the linker. For example, the drug may have an amino, carboxyl, sulfhydryl, hydroxyl, or ketone group that can be bonded to the linker. In the case where the drug is directly linked to the linker, the drug has a reactive active group before linking the antibody.

Useful drug categories include, for example, antitubulin drugs, DNA minor groove binding reagents, DNA replication inhibitors, alkylation reagents, antibiotics, folate antagonists, antimetabolites, chemosensitizers, topoisomerase inhibitors, vinca alkaloids, etc. In the present application, a drug-linker may be used to form an ADC in a simple step. In other embodiments, a bifunctional linker compound may be used to form an ADC in a two-step or multi-step method. For example, a cysteine residue reacts with the reactive portion of the linker in a first step, and in subsequent steps, the functional group on the linker reacts with the drug, thereby forming an ADC.

Usually, the functional group on the linker is selected to facilitate specific reaction with an appropriate reactive group on the drug moiety. As a non-limiting example, an azide-based moiety may be used to react specifically with a reactive alkyne group on the drug moiety. The drug is covalently bonded to the linker by addition of a 1,3-dipole ring between azide and alkyne. Other useful functional groups include, for example, ketones and aldehydes (suitable for reacting with hydrazides and alkoxyamines), and phosphines (suitable for reacting with azides); isocyanates and isothiocyanates (suitable for reacting with amines and alcohols); and activated esters, for example N-hydroxysuccinimidyl esters (suitable for reacting with amines and alcohols). These and other linkage strategies (for example, those described in Bioconjugation Techniques, Second Edition (Elsevier)) are well known to those skilled in the art. Those skilled in the art can understand that, for the selective reaction between the drug moiety and the linker, when a complementary pair of reactive functional groups is selected, each member in the complementary pair may be used both for the linker and for the drug.

The present application further provides a preparation method for an ADC, further comprising: binding an antibody with a drug-linker compound under conditions sufficient to form an antibody conjugate (ADC).

In some embodiments, the method of the present application comprises: binding the antibody to a bifunctional linker compound under conditions sufficient to form an antibody-linker conjugate. In these embodiments, the method of the present application further comprises: binding the antibody-linker conjugate to the drug moiety under conditions sufficient to covalently link the drug moiety to the antibody via the linker.

In some embodiments, the antibody-drug conjugate (ADC) is as shown in the following molecular formula: 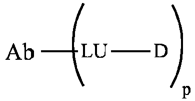 wherein:
AB indicates an antibody,
LU indicates a linker;
D indicates a drug;
and the subscript p is a value selected from 1 to 8.

### Application

The present application provides a use of the antibody of the present application, for example, for the preparation of diagnostic preparations, or the preparation of drugs for the prevention and/or treatment of IL-36-related diseases. The IL-36 related diseases comprise inflammatory diseases, autoimmune diseases, etc., including but not limited to psoriasis, psoriatic arthritis, ankylosing spondylitis, multiple sclerosis, inflammatory bowel disease (such as Crohn's disease and ulcerative colitis), osteoarthritis, rheumatoid arthritis (RA), rheumatic arthritis or osteoporosis, inflammatory fibrosis (e.g., scleroderma, and pulmonary fibrosis and sclerosis), asthma (including allergic asthma), allergic reactions, and cancers.

### Pharmaceutical Composition

The present application further provides a composition. In some cases, the composition is a pharmaceutical composition, which comprises the antigen-binding protein described above, or an active fragment thereof, or a fusion protein thereof, or an ADC thereof, or corresponding CAR-T cells, and a pharmaceutically acceptable carrier. Usually, these substances may be formulated in a non-toxic, inert, and pharmaceutically acceptable aqueous carrier medium, of which the pH is usually about 5-8, for example, or may be about 6-8., although the pH value may vary with the properties of the formulated substances and the disorders to be treated. The formulated pharmaceutical composition may be administered by conventional routes, comprising (but not limited to): intratumoral, intraperitoneal, intravenous, or topical administration.

The antibody of the present application may also be expressed in a cell by means of a nucleotide sequence for cell therapy. For example, the antibody is used for the chimeric antigen receptor T cell immunotherapy (CAR-T), etc.

The pharmaceutical composition of the present application may be directly used to bind an IL-36R protein molecule, and thus may be used to prevent and treat IL-36R-related diseases. In addition, other therapeutic agents may also be used at the same time.

The pharmaceutical composition of the present application comprises the aforementioned monoclonal antibody (or a conjugate thereof) of the present application at a safe and effective amount (such as 0.001-99 wt%, possibly 0.01-90 wt%, or possibly 0.1-80 wt%) and pharmaceutically acceptable carriers or excipients. Such carriers comprise (but are not limited to): saline water, buffer, glucose, water, glycerol, ethanol, and combinations thereof. A pharmaceutical preparation should match the mode of administration. The pharmaceutical composition of the present application may be prepared into an injection form, for example, by means of a conventional method using normal saline or an aqueous solution containing glucose and other adjuvants. The pharmaceutical composition such as an injection and a solution should be manufactured under an aseptic condition. The dosage of an active ingredient is a therapeutically effective amount, for example, about 1 mg/kg body weight to about 5 mg/kg body weight per day. Furthermore, the polypeptide of the present application may also be used together with other therapeutic agents.

When in use, the pharmaceutical composition is administered to mammals at a safe and effective amount, which is usually at least about 10 µg/kg body weight and in most cases, does not exceed about 50 mg/kg body weight. Its dosage may be about 10 µg/kg body weight to about 20 mg/kg body weight. Without doubt, the specific dosage should also consider factors such as the route of administration and the health condition of a patient, which are within the scope of skills of a proficient physician.

### Detection uses and kit

The antibody of the present application can be used in detection applications (for example, for sample detection), so as to provide diagnostic information.

In the present application, the samples (specimens) used comprise cells, tissue samples, and biopsy samples. The term "biopsy" used in the present application shall comprise all types of biopsy known to those skilled in the art. Therefore, the biopsy used in the present application may comprise, for example, tissue samples prepared by an endoscopic method or by organ puncture or needle biopsy.

The samples used in the present application comprise fixed or preserved cell or tissue samples.

The present application further provides a kit comprising the antibody (or a fragment thereof) of the present application. For example, the kit may further comprise a container, an instruction manual, a buffer, etc. For example, the antibody of the present application may be fixed on a detection plate.

### The antibody or fragment thereof involved in the present application may have the following excellent technical effects:

(a) the antibody of the present application has excellent bioactivity and specificity.
(b) Compared with a murine antibody and a chimeric antibody, the humanized antibody of the present application has lower immunogenicity while retaining a comparable affinity with IL-36R.
(c) The blocking of IL-36R by the antibody of the present application can significantly inhibit the inflammatory factor expression pathway in a patient.
(d) The antibody of the present application has a comparable affinity with some non-human mammals (such as monkeys) as with human IL-36R, which facilitates testing and quality control detection in animal models.
(e) The antibody of the present application binds to IL-36R to block the activation signaling pathway for binding IL-36α, IL-36β, or IL-36γ to IL-36R, thereby reducing inflammatory cytokines (such as IL-6, IL-8, or GM-CSF).

The present application is further set forth below in conjunction with specific examples. It should be understood that these embodiments are intended only to illustrating the present application, instead of limiting the scope of the present application. Experimental methods without specific conditions indicated in the following examples usually follow conventional conditions, for example the conditions described in Sambrook et al., Molecular Cloning: A Laboratory Manual (New York: Cold Spring Harbor Laboratory Press, 1989), or according to conditions recommended by the manufacturers. Unless otherwise specified, percentages and parts are weight percentages and parts by weight.

### Examples

### Example 1

The preparation method for murine monoclonal antibodies was based on the hybridoma preparation technology invented by Kohler and Milstein in 1975 (Nature, 1975, 256: 495-497). First, the Fctagged protein (ACRO, #IL2-H5254) of human IL-36R was emulsified with the Freund's adjuvant, and then used to immunize multiple strains of mice. After four rounds of immunizations, the serum was collected to detect the titer by ELISA, and spleen cells were harvested from the selected optimal mice for fusion with SP2/0 myeloma cells. After HAT screening of hybridoma polyclonal cells, a polyclonal culture supernatant was tested in the binding activities of human IL-36R, monkey IL-36R and human IL1R1, and the functional inhibition effects of polyclonal antibodies on IL-36 stimulating factors was detected. The best polyclones were picked for monoclonalization, the resulting monoclones were also screened in binding activity and functional activity, and the affinity was detected by the Biacore method to finally select the best hybridoma monoclonal cell strains for sequence analysis.

**Table 1. Partial data of hybridoma screening**

| Clone name | Binding activity OD450 value | MFI ratio in binding activity to human IL-36R (hybridoma supernatant/p ositive control) | MFI ratio in binding activity to monkey IL-36R (hybridoma supernatant/p ositive control) | MFI ratio in binding activity to human IL1R1 (hybridoma supernatant/n egative control) | IL-6 ratio in blockage of human IL36β functional activity (hybridoma supernatant/p ositive control) | IL-8 ratio in blockage of human IL36α functional activity (hybridoma supernatant/p ositive control) | IL-8 ratio in blockage of human IL36β functional activity (hybridoma supernatant/p ositive control) | IL-8 ratio in blockage of human IL36 γ functional activity (hybridoma supernatant/p ositive control) |
|---|---|---|---|---|---|---|---|---|
| 6(1)-B8 | 1.293 | 75.66% | 94.41% | 120.18% | 10.50% | 4.78% | 5.08% | 5.77% |
| 6(2)-G8 | 1.74 | 94.54% | 81.50% | 114.91% | 13.18% | 5.61% | 6.45% | 5.58% |
| 21(1)-E4 | 0.943 | 67.89% | 90.03% | 122.81% | 5.70% | -3.19% | -1.97% | -2.50% |
| 21(2)-F3 | 1.681 | 80.91% | 91.36% | 114.91% | 1.18% | -2.61% | -1.88% | -2.31% |
| 65(1)-B5 | 1.355 | 56.84% | 85.44% | 111.40% | 23.05% | 15.66% | 18.02% | 12.87% |
| 65(2)-F11 | 1.319 | 71.60% | 88.52% | 116.67% | 21.66% | 15.90% | 17.72% | 13.17% |
| 74(1)-E2 | 1.917 | 109.76% | 85.93% | 114.04% | 1.00% | -3.11% | -3.15% | -2.89% |
| 74(2)-GS | 1.827 | 103.89% | 97.44% | 114.91% | 2.84% | -2.86% | -2.66% | -2.60% |
| 79(1)-C7 | 2.133 | 112.13% | 100.76% | 110.53% | 13.08% | 3.12% | 3.51% | 3.34% |
| 79(2)-G7 | 1.085 | 75.48% | 102.59% | 121.93% | 8.75% | 4.70% | 1.46% | 3.24% |
| 84(1)-C11 | 2.277 | 84.52% | 86.85% | 115.79% | 0.90% | -3.27% | -3.35% | -2.99% |
| 84(2)-C3 | 2.324 | 64.61% | 104.35% | 114.91% | 0.81% | -4.02% | -3.05% | -3.47% |
| 111(1)-E5 | 2.161 | 81.64% | 105.10% | 118.42% | 0.07% | -4.02% | -4.62% | -3.86% |
| 111(2)-B3 | 2.36 | 102.30% | 103.62% | 116.67% | 0.07% | -4.68% | -4.62% | -4.06% |
| 130(1)-H7 | 1.316 | 113.29% | 110.61% | 119.30% | 30.89% | 44.55% | 26.64% | 25.52% |
| 130(2)-A2 | 1.507 | 83.19% | 130.12% | 123.68% | 31.35% | 43.22% | 27.13% | 22.31 % |
| 140(1)-C1 | 1.768 | 84.57% | 102.87% | 117.54% | 1.92% | -4.35% | -3.74% | -3.09% |
| 172(1)-H4 | 1.442 | 95.40% | 96.88% | 114.91% | 44.92% | 58.91 % | 46.82% | 68.93% |
| 172(2)-C4 | 1.814 | 82.50% | 89.66% | 116.67% | 45.10% | 55.75% | 46.73% | 67.56% |
| 179(1)-G7 | 1.667 | 74.17% | 113.09% | 114.04% | 30.06% | 27.19% | 24.48% | 18.32% |
| 180(1)-H5 | 2.082 | 81.42% | 79.75% | 116.67% | 9.48% | 0.21% | 1.46% | -1.63% |
| 180(2)-D3 | 2.477 | 65.03% | 86.87% | 114.91% | 13.54% | 3.54% | 4.89% | 1.78% |
| 181(2)-F10 | 2.959 | 49.74% | 109.08% | 126.32% | 20.65% | 15.07% | 12.53% | 10.15% |
| 182(1)-C8 | 1.843 | 93.33% | 86.26% | 120.18% | 50.27% | 74.76% | 57.11% | 55.89% |
| 183(1)-H7 | 2.016 | 84.65% | 84.83% | 115.79% | 30.89% | 26.86% | 23.99% | 22.31 % |
| 183(2)-F12 | 1.857 | 59.34% | 84.47% | 127.19% | 24.71% | 18.23% | 18.90% | 14.53% |
| 184(1)-F8 | 2.27 | 96.60% | 86.57% | 118.42% | 0.26% | -3.60% | -5.11% | -4.06% |
| 184(2)-B9 | 2.408 | 98.36% | 89.91% | 120.18% | -1.80% | -4.45% | -7.19% | -3.44% |

As shown in Table 1, after extensive hybridoma screening, the antibody expression supernatants of hybridomas 6(1)-B8 and 111(1)-E5 showed very high binding activity to human IL-36R and monkey IL-36R proteins at a cell binding level, but no specific binding to human IL1R1 proteins, and showed an obvious functional blocking effect on cytokines produced by the stimulation of IL-36 stimulating factors (IL-36α, IL-36β, and IL-36γ).

### Example 2 Cloning and humanization of variable region gene sequences of anti-IL-36R antibodies

### 2.1 Cloning of antibody variable region genes in hybridoma cells

Based on the principle of TAKARA's 5' RACE technology, the cDNA sequences of the variable regions of the mouse antibody expressed by the hybridoma cell strains were cloned. Briefly, the variable region gene-specific cDNAs of the heavy and light chains were synthesized by using the SMARTer 5'RACE synthesis kit (TAKARA, Cat. #:634859) following the instructions. The 5'- and 3'-terminals of the cDNA sequences were modified with PCR primers, which were designed to add appropriate leader sequences to the variable region cDNAs of the heavy and light chains, respectively, such that the resulting PCR products could be cloned, by a seamless cloning method, onto a heavy-chain vector pHB-Fc and a light-chain vector pHB-Cκ which are expressed in the existing recombinant antibody. The expression vector pHB-Fc contained the human IgG1 heavy-chain constant region gene sequence, with L234A and L235A (Eu numbering) mutations having weakened antibody ADCC effects on CH2; and the vector pHB-Cκ contained the human κ light-chain constant region gene sequence. The PCR amplification products of the heavy- and light-chain variable regions were cloned into the expression vectors by means of an In-fusion cloning reagent (TAKARA, Cat. #: 639650) to obtain human-mouse chimeric antibody expression vectors, which were then transformed into *E. coli* DH5α competent cells (Yeastern Biotech, Cat. #: FYE607-80VL). Monoclonal colonies were picked for Sanger sequencing, and antibody variable region sequences were obtained by analysis. As a result, anti-IL-36R chimeric antibodies (HUABO No.: 900497) was obtained, with the light chain derived from the hybridoma 6(1)-B8 in Example 1, and the heavy chain derived from the hybridoma 111(1)-E5 in Example 1. The sequences of its variable regions were as follows:
900497 HCVR SEQ ID NO: 1 
QVQLQQTGSVLVRPGTSVKLSCKASGYTFTSSWMHWAKQRPGQGLEWIGEIHPNSAKTNY
NEKFKGKATLTVDTFSSTAYVDLSSLASEDSAVYYCARVDYGKPWFAYWGQGTLVTVSA
900497 LCVR SEQ ID NO: 2

QIVLTQSPTIMSASPGERVTMTCSASSSVSSSYLHWYQQKPGSSPKLWIYSTSNLASGVPARF
SGSGSGTSYSLTINSMEAEDAATYYCQQFQSSPLTFGAGTKLGLK

Here, the underlined sections were CDRs (as defined by IMGT, the single sequences were as follows):

**Table 2. CDR sequences of murine anti-IL-36R antibody**

| Domain | | Sequence | SEQ ID NO: |
|---|---|---|---|
| VH | CDR1 | GYTFTSSW | 3 |
| | CDR2 | IHPNSAKT | 4 |
| | CDR3 | ARVDYGKPWFAY | 5 |
| VL | CDR1 | SSVSSSY | 6 |
| | CDR2 | STS | 7 |
| | CDR3 | QQFQSSPLT | 8 |

### 2.2 Expression of chimeric antibodies

The expression vectors obtained in 2.1 were amplified by means of *Escherichia coli*, and a sufficient amount of plasmids were prepared using an endotoxin-free plasmid extraction kit (Tiangen Biotech (Beijing) Co., Ltd., Cat. #: DP117) for transient transfection to express the chimeric antibody. CHO-S cells (ThermoFisher, Cat. #: R80007) were used as host cells for expression. The two prepared heavy-chain vectors, respectively with the light-chain vectors, were mixed with polyetherimide (PEI, Polysciences, Cat. #: 24765-1) to form liposome complexes for transfecting the CHO-S cells, which were then cultured for 5-7 days in a carbon dioxide shaker. The cell culture supernatant was collected through centrifugation, and purified by a Protein A affinity chromatographic column to obtain a human-mouse chimeric antibody.

### 2.3 Humanization of murine anti-human IL-36R antibody: preparation method for humanized antibodies

The antibodies were humanized by aligning the variable region sequence of the chimeric antibody (HUABO Number: 900497) with available sequences in the NCBI IgBlast database, and carrying out identification and analysis to finally determine a human framework region (FR) suitable for constructing CDR-grafted heavy and light chains thereon.

During transformation, transformation sites were designed according to the conserved amino acid residues in the FR of the human antibody and the important amino acid residues in the FR of the antibody; the variable regions of the heavy and light chains of the chimeric antibodies were respectively designed for humanized mutations; and humanized point-mutation antibody expression plasmids were amplified and constructed using PCR. The humanized point-mutation antibody expression plasmids were expressed in the CHO-S cells and then purified respectively to obtain humanized antibody proteins. With Biacore and methods for detecting cell bioactivity and the like, the affinity of humanized antibodies, activated cytokine release and other indicators were screened to obtain three humanized anti-IL-36R antibodies with excellent performance. The resulting humanized anti-IL-36R antibodies were numbered as 900513, 900527, and 900534, and their VH and VL sequences were shown as follows:
900513, 900527 and 900534 HCVR SEQ ID NO: 9
900513 LCVR SEQ ID NO: 10
900527 LCVR SEQ ID NO: 11
900534 LCVR SEQ ID NO: 12

QIVLTQSPGTLSLSPGERATLSCRASSSVSSSYLHWYQQKPGQAPRLLIYSTSSRATGIPDRFS
GSGSGTDFTLTISRLEPEDFATYYCQQFQSSPLTFGQGTKLEIK

Here, the underlined sections were CDRs (defined according to IMGT), and 900513, 900527, and 900534 were the numbers for the three humanized antibody proteins respectively. The CDRs of humanized antibodies 900513, 900527 and 900534 were the same as those of the chimeric antibody 900497, and the FRs of humanized antibodies were mutated on the basis of chimeric 900497.

### Example 3 Detection of bioactivity of anti-human IL-36R humanized antibodies

### 3.1 Biacore (SPR) for detecting antibody affinity

Test materials and instruments were as follows:
His-tagged human IL-1 Rrp2/IL-1 R6 protein (Human IL-1 Rrp2/IL-1 R6 Protein, His Tag), ACRO Biosystems, IL2-H52H6
HBS-EP+ (10X), GE, BR-1006-69
Series S Sensor Chip CM5, GE, 29-1275-56
His Capture Kit, GE, 28995056
BIACORE, GE, Biacore 8K
Glycine solution at pH 1.5, GE, BR100354
The test method was as follows.

Series S Sensor Chip CM5 was equilibrated for 20-30 min at room temperature, and then fitted in the instrument. Following the instruction manual for His Capture Kit, an anti-His antibody was immobilized onto the Series S Sensor Chip CM5. An HBS-EP+ (10X) solution was diluted 10-fold in ultrapure water as the running buffer. An antigen (His-tagged human IL-1 Rrp2/IL-1 R6 protein) was diluted to 1 µg/ml and 10 µL/min using the running buffer, and loaded during 15 sec to capture around 50 RU of antigen. An antibody sample were diluted to 30 nM, 15 nM, 7.5 nM, 3.75 nM, 1.875 nM, 0.9375 nM, and 0.46875 nM using an equilibration buffer; the running buffer was taken as zero concentration; at 30 µL/min, binding and dissociation were allowed to occur for 120 s and 900 s, respectively; and the chip was regenerated with the glycine solution at pH 1.5. The sample was analyzed by a capture-based single-cycle kinetics program; the corresponding analysis program was selected to analyze the data, and it was confirmed that there was no obvious reference binding; and the Kinetics 1:1 binding model was selected for fitting analysis to acquire the kinetic parameters of the sample.

**Table 3 Affinity assay of candidate anti-human IL-36R humanized antibodies**

| Protein number | ka (1/Ms) | kd (l/s) | KD (M) |
|---|---|---|---|
| 900513 | 9.04E+05 | 1.34E-05 | 1.48E-11 |
| 900527 | 9.67E+05 | 4.37E-05 | 4.52E-11 |
| 900534 | 8.81E+05 | 5.10E-05 | 5.79E-11 |
| 900497 | 6.60E+05 | 1.68E-05 | 2.54E-11 |
| 900389 | 7.39E+05 | 1.17E-05 | 1.58E-11 |

Note: 900389, as an anti-human IL-36R antibody developed by BI, was obtained through independent expression by HUABO who synthesized a variable region gene from sequences recorded according to the patent WO2013/074569A1. Specifically, the heavy-chain variable region of 900389 was 81B4vH33_90vH as set forth in SEQ ID NO: 89 in WO2013/074569A1, the light-chain variable region of 900389 was 81B4vK32_105vK as set forth in SEQ ID NO: 77 in WO2013/074569A1, and both 81B4vH33_90vH and 81B4vK32_105vK were obtained by carrying out humanization on the basis of a murine antibody 81B4; and the constant region of 900389 was the same as those of the candidate antibodies of the present application.

The affinity constant (KD(M)) for the binding of each humanized antibody to human IL-1 Rrp2 was shown in Table 3. The results showed that the affinity of the humanized monoclonal antibodies of the present application were close to the order of magnitude of 10⁻¹¹, showing a very strong affinity.

### 3.2 Detection of functional inhibition effect of anti-human IL-36R antibody on huIL-36 stimulating factor

A human ovarian cancer cell line NCI/ADR-RES was added to a 96-well cell culture plate at 100 µL per well (4.5×10⁴ cells per well), and incubated overnight at 37°C with 5% CO₂; a series of samples to be detected was diluted and added at 50 µL/well; the plate was incubated for 15 min at 37°C with 5% CO₂; ligands huIL-36α (1 µg/mL), huIL-36β (80 µg/mL), and huIL-36γ (120 µg/mL) were added at 50 µL/well, respectively, and mixed well; and the plate was incubated for 18-24 h at 37°C with 5% CO₂. Cell culture supernatants were obtained through centrifuging and detected with the Biolegend ELISAkit as follows: huIL-6 (diluted at 1:100, Cat. #: 430503), huIL-8 (diluted at 1:5, Cat. #: 431503), and huGM-CSF (diluted at 1:2, Cat. #: 432003).The activation of NF-κB in NCI/ADR-RES cells was tested under huIL-36β stimulation as follows: the ligand huIL-36β was diluted to 240 ng/mL, added to cells pre-incubated with the antibody to be detected, and incubated for 30 min at 37°C with 5% CO₂. Supernatants were obtained through centrifugation, and after cell lysis, lysate was taken and subjected to NF-xB phosphorylation assay with a kit (Cisbio, Cat. #: 64NFBPEG).

**Table 4 Results of bioactivity detection of candidate humanized antibodies**

| Stimulating factor | Detection index /IC50 (ng/mL) | 900389 | 900513 | 900527 | 900534 | 900497 |
|---|---|---|---|---|---|---|
| IL-36 α | IL-6 | 21.85 | 12.40 | 15.03 | 21.58 | 33.22 |
| | IL-8 | 57.45 | 39.78 | 31.53 | 30.39 | 43.91 |
| | GM-CSF | 33.17 | 20.65 | 22.00 | 20.17 | 32.83 |
| IL-36 β | IL-6 | 24.59 | 22.34 | 13.92 | 16.80 | 20.39 |
| | IL-8 | 34.44 | 22.09 | 22.27 | 21.97 | 29.21 |
| | GM-CSF | 26.91 | 18.40 | 17.36 | 18.65 | 22.36 |
| IL-36 γ | IL-6 | 26.44 | 12.05 | 16.18 | 17.61 | 19.78 |
| | IL-8 | 33.71 | 18.00 | 19.81 | 17.95 | 28.37 |
| | GM-CSF | 32.67 | 17.87 | 19.81 | 20.97 | 28.75 |
| IL-36 β | pNF-κB | 179.00 | 105.30 | 71.44 | 64.92 | 115.10 |

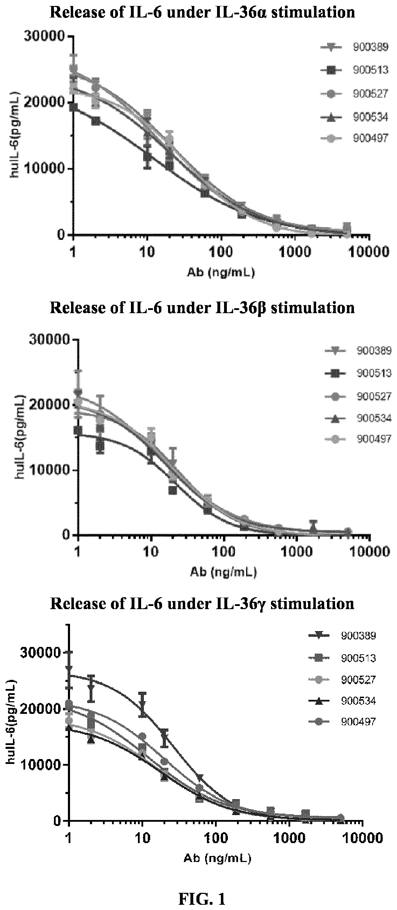

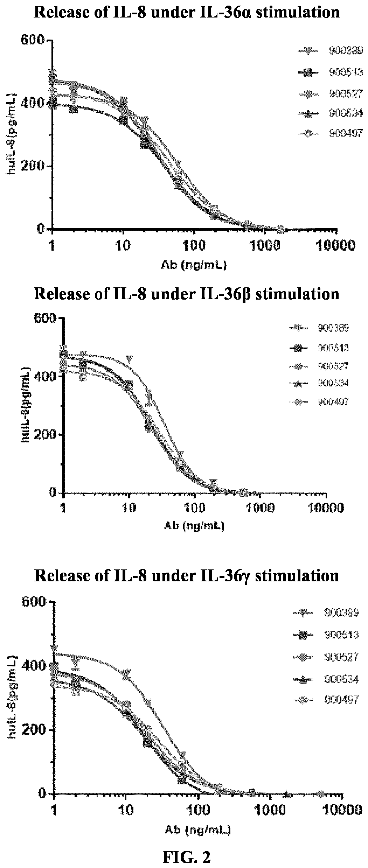

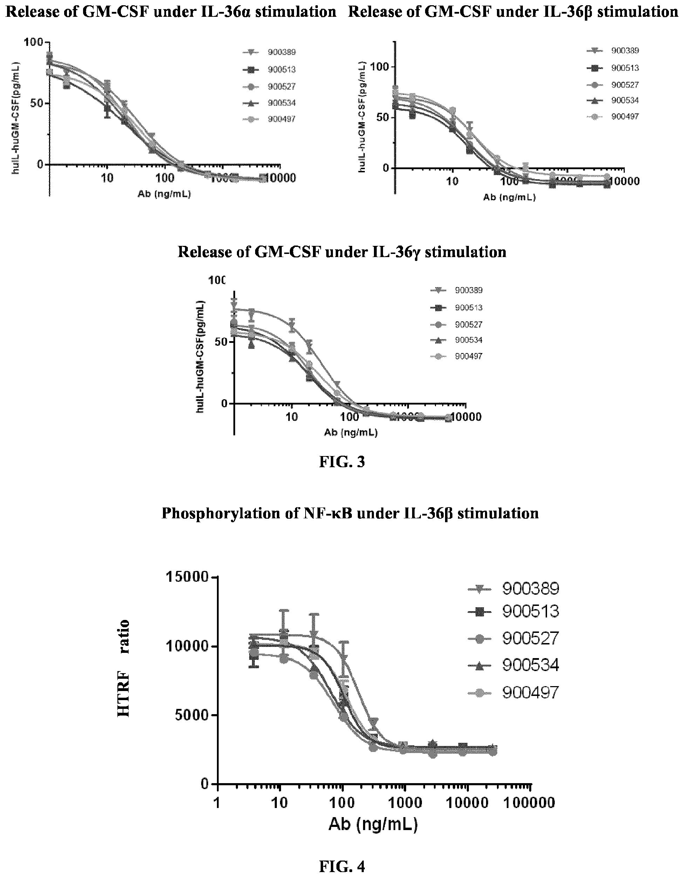

The results were shown as in FIG. 1 to FIG. 4 and Table 4. The humanized antibodies 900513, 900527, and 900534 showed a significant blocking effect on the function of human stimulating factors IL-36α, IL-36β and IL-36γ, such that the secretion of cytokines IL-6, IL-8 and GM-CSF and the phosphorylation of NF-κB could be significantly blocked.

### Example 4 Non-specific binding experiment (SPR) of chimeric antibodies and humanized antibodies

In this test, the SPR method was used to determine the non-specific adsorption effect between antibodies and non-target molecules, and the instrument and equipment as well as reagent materials used in the test were shown as in Table 5 and Table 6.

**Table 5 Instrument and equipment**

| Designation | Factory | Model | No. |
|---|---|---|---|
| Biacore | GE | Biacore 8K | 1305 |

**Table 6 Reagent materials:**

| Designation | Manufacturer/ Source | Catalog No. | Batch No. |
|---|---|---|---|
| Series S Sensor Chip CM5 | GE | BR-1005-30 | 10253038 |
| Egg-derived lysozyme solution | Sigma | L3790 | SLBM3565V |
| Soybean-derived trypsin inhibitor type 1-S | Sigma | T-2327 | SLBJ6832V |
| Polyclonal rabbit anti-lysozyme | ABcam | Ab391 | GR215714-19 |
| Anti-trypsin inhibitor antibodies | LifeSpan Biosciences | LS-C76609 | 26978 |

The Series S Sensor Chip CM5 (GE, #BR-1005-30) was equilibrated for 20-30 min at room temperature and fitted into the Biacore 8K (GE) instrument. The egg-derived lysozyme solution (Sigma, #L3790) and the soybean-derived trypsin inhibitor type 1-S (Sigma, #T-2327) were immobilized to the CM5 chip using an amino conjugation kit (GE, #BR-1000-50). HBS-EP(1X) (GE, #BR-1006-69) was used as the loading buffer and 4 equilibrium cycles were set. The polyclonal rabbit anti-lysozyme (ABcam, Ab391), the anti-trypsin inhibitor antibody (LifeSpan Biosciences, #LS-C76609), the chimeric antibody, and the humanized antibody were diluted to 1000 nM with the equilibration buffer, respectively; and the flow rate was set to 5 µL/min, loading channels to 1, 2 and 3, and Flow Cells to 1 and 2. The binding time was 10 min and the dissociation time was 15 min. At the regeneration flow rate of 50 µL/min, the regeneration was carried out first with 0.85% phosphoric acid solution (ProteOn, 176-2260) for 60 s, and then with 50 mM sodium hydroxide solution for 30 s. Here, the sample 900389, as an anti-human IL-36R humanized antibody developed by BI, was obtained through independent expression by HUABO who synthesized variable region genes according to the patent. The sample 900497 was an anti-IL-36R chimeric antibody constructed through hybridoma screening by HUABO.

The results showed that the binding signal of 900389 to the egg-derived lysozyme solution was greater than 20 RU, as shown in Table 7. It was thus believed that the non-specific electrostatic and hydrophobic binding action existed The binding signal of 900497 to the egg-derived lysozyme solution and the binding signal of 900497 to the soybean-derived trypsin inhibitor type 1-S were both less than 20. It was thus believed that the non-specific electrostatic and hydrophobic binding action did not exist.

**Table 7 Comparison of non-specific binding of samples**

| Designation | Lysozyme (RU) | Trypsin inhibitor (RU) | Deactivated carboxymethyl glucan (RU) | Carboxymethyl glucan (RU) |
|---|---|---|---|---|
| Buffer | 24.0 | 12.9 | 16.2 | 14.0 |
| Polyclonal rabbit anti-lysozyme | 9316.9 | 32.2 | 34.3 | 27.7 |
| Anti-trypsin inhibitor antibodies | 65.1 | 6259.1 | 36.8 | 22.5 |
| 900389 | 55.0 | 27.5 | 16.3 | 14.2 |
| 900497 | 24.3 | 16.7 | 16.4 | 14.4 |

| | | | | |
|---|---|---|---|---|
| Note: It was generally believed that after the influence of buffer was deducted, the response value below 20 RU indicated a weak interaction which was ignorable, the response value greater than 20 RU indicated a significant interaction; and the response value above 100 RU indicated a strong interaction. | | | | |

### Example 5 Cell binding and function experiment of stable cell line antibodies

The BI control sequence 900389, the humanized sequence 900527 screened by HUABO, and TNP IgG1 (Fc silence) 900543 were inserted into HUABO GS vectors to construct recombinant expression plasmids for transfecting CHO-K1 cells, respectively, thereby constructing stable cell lines. The stable cell line constructed from HUABO 900527 has a protein number of HB0034, and the screened monoclonal cell lines were optimized by the upstream process to achieve the expression level of greater than 4 g/L. The antibodies expressed by stable cell lines were purified and then tested by flow cytometry binding experiments and functional experiments. The sample 900543 was a TNP IgG1 (Fc silence) negative control antibody.

### 5.1 Flow cytometry binding experiment for stable cell line antibodies

The binding activity of the stable cell line antibodies to human IL-36R and monkey IL-36R was detected, and the cell lines for flow cytometry detection were CHO-K1 cell lines that were constructed internally by HUABO and expressed human IL-36R and monkey IL-36R.

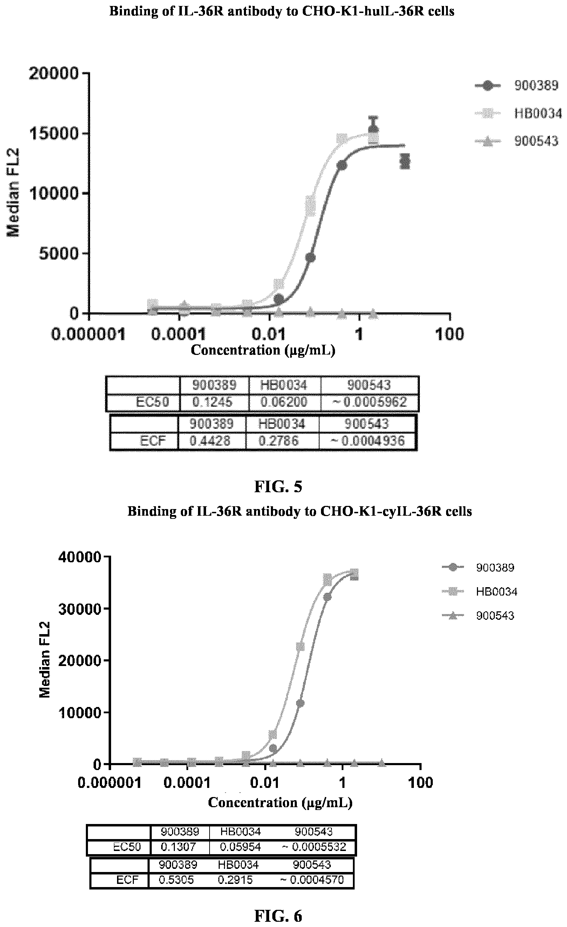

The detection results were shown as in Table 8 and FIG. 5 and FIG. 6.

**Table 8 Detection of binding activity of antibodies to human IL-36R and monkey IL-36R**

| Name of antibody | Binding activity to human IL-36R EC50 (ug/mL) | Binding activity to human IL-36R EC90 (ug/mL) | Binding activity to monkey IL-36R EC50 (ug/mL) | Binding activity to monkey IL-36R EC90 (ug/mL) |
|---|---|---|---|---|
| 900389 | 0.1245 | 0.4428 | 0.1307 | 0.5305 |
| HB0034 | 0.062 | 0.2786 | 0.05954 | 0.2915 |
| 900543 | No binding | No binding | No binding | No binding |

The results showed that HUABO monoclonal antibody HB0034 had high binding activity to both human IL-36R and monkey IL-36R. Compared with the positive control antibody 900389, the antibody of the present application had lower EC50 value and showed stronger binding activity to human IL-36R.

### 5.2 Experiments on detection of functional inhibition effect of stable cell line antibodies on huIL-36 stimulating factor

5.2.1 Functional inhibition effect of antibodies on huIL-36 stimulating factor in NCI/ADR-RES cells The human ovarian cancer cell line NCI/ADR-RES was added to a 96-well cell culture plate at 100 µL per well (4.5×10⁴ cells per well), and incubated overnight at 37°C with 5% CO₂; a series of samples to be tested was diluted and then added at 50 µL/well, incubated for 15 min at 37°C with 5% CO₂; the ligand huIL-36β (40 ng/mL) was added at 50 µL/well, mixed thoroughly, and cultured for 18-24 h at 37°C with 5% CO₂. The cell culture supernatant was obtained through centrifuging, and tested in huIL-6 with the HTRF kit (Cat. #: 62HIL06PEG, CISBIO).

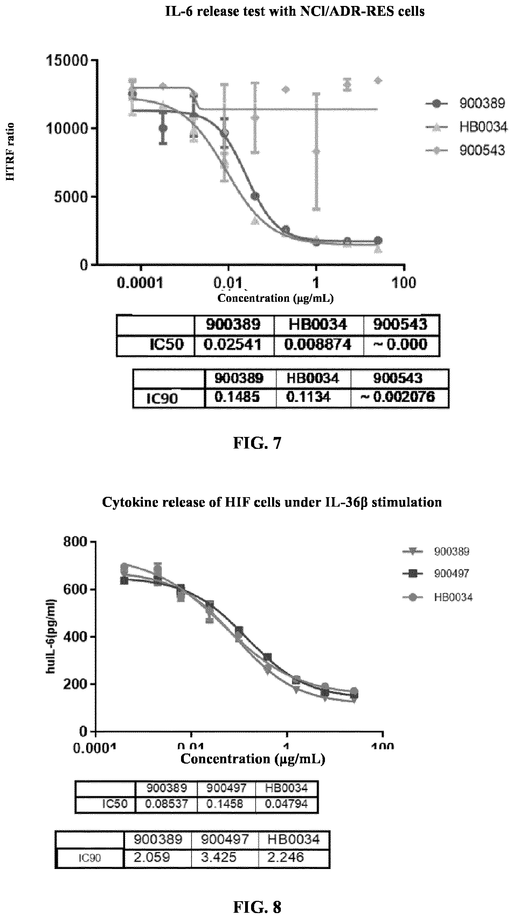

The results were shown as in Table 9 and FIG. 7.

**Table 9 Functional inhibition effect of antibodies on huIL-36 stimulating factor in NCI/ADR-RES cells**

| Name of antibody | HuIL-6 detected in NCI/ADR-RES under IL-36β stimulation IC50 (ug/mL) | HuIL-6 detected in NCI/ADR-RES under IL-36β stimulation IC90 (ug/mL) |
|---|---|---|
| 900389 | 0.02541 | 0.1485 |
| HB0034 | 0.008874 | 0.1134 |
| 900543 | No effect | No effect |

The results showed that the humanized antibody HB0034 of the stable cell line had a significant blocking effect on the function of the human stimulating factor IL-36β, and could significantly block the secretion of cytokine IL-6. Compared with the positive control antibody 900389, the antibody of the present application had a lower IC50 value and a stronger blocking effect.

### 5.2.2 Functional inhibition effect of antibodies on huIL-36 stimulating factor in HIF cells

Human intestinal fibroblasts (HIF) cells were prepared into a 4.5E5/ml cell suspension with a Serum Starvd medium, added to a 96-well plate at 100 ul (45,000 cells) per well, and incubated overnight. The series of antibodies was diluted, added at 50 µl/well, and incubated for 15 min at 37°C. IL-36α was diluted to 1000 ng/ml, added at 50 µl per well, and mixed thoroughly. The mixture was incubated for 4 h at 37°C with 5% CO₂, and centrifuged to collect a supernatant, in which the content of human IL-6 was detected. IL-36β was diluted to 80 ng/m, added at 50 µl per well, and mixed thoroughly. The mixture was incubated for 4 h at 37°C with 5% CO₂, and centrifuged to collect a supernatant, in which the content of human IL-6 was detected. IL-36β was diluted to 40 ng/m, added at 50 µl per well, and mixed thoroughly. The mixture was incubated for 20 min at 37°C with 5% CO₂, and the content of pNFκB was detected using the kit.

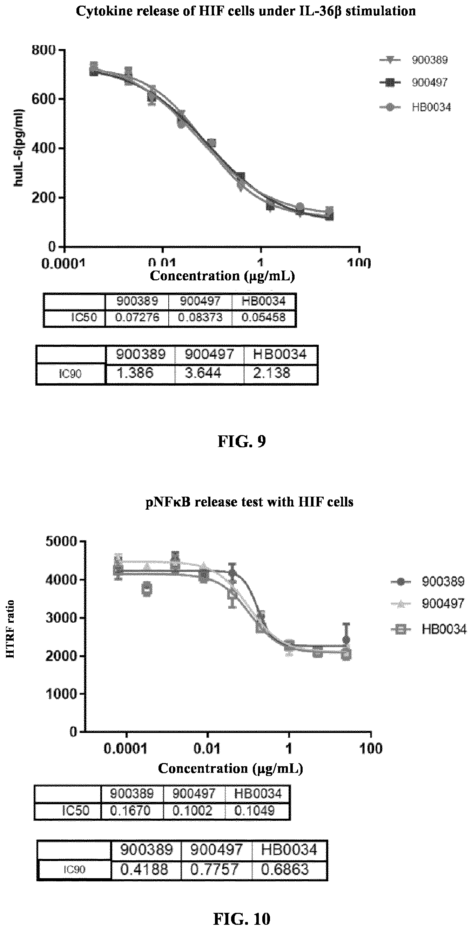

The results were shown as in Table 10 and FIG. 8, Table 11 and FIG. 9, Table 12 and FIG. 10.

**Table 10 Functional inhibition effect of antibodies on huIL-36 stimulating factor in HIF cells**

| Name of antibody | HuIL-6 detected in HIF under IL-36α stimulation IC50 (ug/mL) | HuIL-6 detected in HIF under IL-36α stimulation IC90 (ug/mL)nL) |
|---|---|---|
| 900389 | 0.08537 | 2.059 |
| 900497 | 0.1458 | 3.425 |
| HB0034 | 0.04794 | 2.246 |

**Table 11 Functional inhibition effect of antibodies on huIL-36 stimulating factor in HIF cells**

| Name of antibody | HuIL-6 detected in HIF under IL-36β stimulation IC50 (ug/mL) | HuIL-6 detected in HIF under IL-36β stimulation IC90 (ug/mL) |
|---|---|---|
| 900389 | 0.07276 | 1.386 |
| 900497 | 0.08373 | 3.644 |
| HB0034 | 0.05458 | 2.138 |

**Table 12 Functional inhibition effect of antibodies on huIL-36 stimulating factor in HIF cells**

| Name of antibody | pNFκB detected in HIF under IL-36β stimulation IC50 (ug/mL) | pNFκB detected in HIF under IL-36β stimulation IC90 (ug/mL) |
|---|---|---|
| 900389 | 0.1670 | 0.4188 |
| 900497 | 0.1002 | 0.7757 |
| HB0034 | 0.1049 | 0.6863 |

The results showed that the humanized antibody HB0034 of the stable cell line had a significant blocking effect on the function of human stimulating factors IL-36α and IL-36β, and could significantly block the secretion of cytokine IL-6 and the phosphorylation of NF-κB. Compared with the positive control antibody 900389, the antibody of the present application had a lower IC50 value and a stronger blocking effect.

### Example 6 Non-specific binding experiment (SPR) of humanized antibodies

In this test, the SPR method was used to determine the non-specific adsorption effect between antibody samples (900389 and HB0034) and non-target molecules, and the reagent materials used in the test were shown as in Table 13.

**Table 13 Reagent materials**

| Designation | Manufacturer/S ource | Catalog No. | Batch No. |
|---|---|---|---|
| Series S Sensor Chip CM5 | GE | BR-1005-30 | 10253038 |
| Lysozyme solution from chicken egg | Sigma | L3790 | SLBM3565V |
| Trypsin inhibitor type 1-S from Glycine max | Sigma | T-2327 | SLBJ6832V |
| Polyclonal rabbit anti-lysozyme | Abeam | Ab391 | GR215714-19 |
| Anti-trypsin inhibitor antibody | LifeSpan Biosciences | LS-C76609 | 26978 |

The Series S Sensor Chip CM5 (GE, #BR-1005-30) was equilibrated for 20-30 min at room temperature and fitted into the Biacore 8K (GE) instrument. The egg-derived lysozyme solution (Sigma, #L3790) and the soybean-derived trypsin inhibitor type 1-S (Sigma, #T-2327) were immobilized to channels 1 and 2 of the CM5 chip using an amino conjugation kit (GE, #BR-1000-50). HBS-EP+(1X) (GE, #BR-1006-69) was used as the running buffer and 5 equilibrium cycles were set. The polyclonal rabbit anti-lysozyme (ABcam, #Ab391), the anti-trypsin inhibitor antibody (LifeSpan Biosciences, #LS-C76609), the chimeric antibody, and the humanized antibody were diluted to 1000 nM with the running buffer, respectively; and the flow rate was set to 5 µL/min, loading channels to 1, 2 and 3, and Flow Cells to 1 and 2. The binding time was 10 min and the dissociation time was 15 min. At the regeneration flow rate of 50 µL/min, the regeneration was carried out first with 0.85% phosphoric acid solution (BIO-RAD, #176-2260) for 60 s, and then with 50 mM sodium hydroxide solution (GE, #BR-1003-58) for 30 s.

The results showed that, as shown in Table 14, the binding response value of the BI control antibody 900389 to lysozyme was greater than 20 RU and its binding response value to trypsin was less than 20 RU, indicating that the samples had negative charges. In this experiment, the binding response value of 900389 to carboxymethyl glucan was 53.9 RU, indicating that the samples were capable of binding to the carboxymethyl glucan. The binding response value of HB0034 to lysozyme and the binding response value of HB0034 to trypsin were both less than 20 RU, and the sample could be considered showing no obvious non-specific electrostatic binding effect.

**Table 14 Comparison of non-specific binding of samples**

| Designation | Lysozyme (RU) | Trypsin inhibitor (RU) | Deactivated carboxymethyl glucan (RU) | Carboxymethyl glucan (RU) |
|---|---|---|---|---|
| Buffer | 0 | 0 | 0 | 0 |
| Polyclonal rabbit anti-lysozyme | 5554.4 | 15.1 | -8.5 | 18.0 |
| Anti-trypsin inhibitor antibodies | 17.5 | 9920.5 | -7.3 | 14.6 |
| 900389 (BI control antibody) | 21.6 | 14.6 | 0.3 | 53.9 |
| HB0034 | 12.1 | 12.0 | -1.3 | -2.4 |

| | | | | |
|---|---|---|---|---|
| Note: It was generally believed that after the influence of buffer was deducted, the response value below 20 RU indicated a weak interaction which was ignorable, the response value greater than 20 RU indicated a significant interaction; and the response value above 100 RU indicated a strong interaction. | | | | |

### Example 7 Therapeutic effect of humanized antibodies on psoriasis

Cynomolgus monkeys were assigned into five groups (with six monkeys in each group), namely a model control group, a positive control group, a low-dose HB0034 group, a mid-dose HB0034 group, and a high-dose HB0034 group. A model of psoriasis was established by applying imiquimod (IMQ) cream. From the day of first modeling (Day 0) and on the 4th day (Day 4), the low-, mid- and high-dose HB0034 groups of were administered with intravenous injections of 5, 15, and 50 mg/kg HB0034 respectively; the model control group was administered with normal saline; and the positive control group was administered with dexamethasone (5 mg/cm², applied once a day).

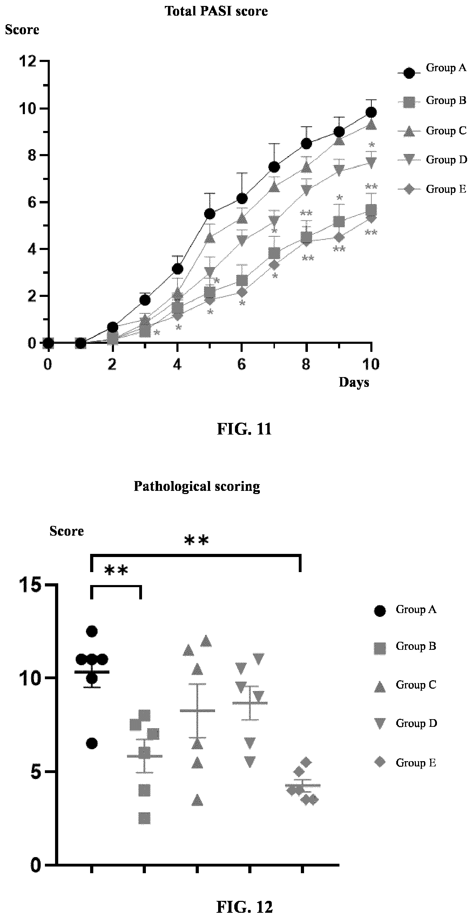

The experimental results showed that at the end of the test, the weight of each group decreased slightly, and the weight loss in the HB0034 treatment groups decreased with the increase of dose. Compared with normal saline (model group), HB0034 at a dose of 5 mg/kg significantly reduced the levels of IL-17 and IL-36 (p<0.05) in molded skin tissues, showing a trend towards improvement in dermatopathological scoring, but the difference was not significant (p > 0.05) (see Table 15); HB0034 at a dose of 15 mg/kg significantly improved the PASI scoring of the model (Day 10 as experimental endpoint, p<0.05), showing a trend towards improvement in dermatopathological scoring, but the difference was not significant (p > 0.05) (see Table 15, FIG. 12), and the levels of IL-17 and IL-36 in the molded skin tissues were significantly reduced (p < 0.05); and HB0034 at a dose of 50 mg/kg significantly improved the PASI scoring of the model (Days 4 to 10, p < 0.05), and significantly reduced the dermatopathological scoring and the levels of skin IL-17 and IL-36 (p < 0.05) (see Table 15, FIG. 11 to FIG. 12). Here, in FIG. 11, n = 6, ^{∗} p < 0.05, ^{∗∗} p < 0.01 v.s. normal saline; and in FIG. 12, n=6, ^{∗∗} p < 0.01 v.s. normal saline.

In this mode, the efficacy of the positive control drug dexamethasone (5 mg/cm²) was slightly better than that of 15 mg/kg HB0034 and slightly weaker than that of 50 mg/kg HB0034.

It can be seen that HB0034 at a dose of 15-50 mg/kg (i.v., Q4D × 2) alleviates the IMQ-induced psoriasis-like symptoms such as skin erythema, dander, and skin thickening, reduces the PASI scoring, improves the dermatopathological scoring at the modeled sites, and reduces the levels of IL-17 and IL-36 in skin tissues, in a dose-dependent manner. HB0034 has a minimum effective dose of 15 mg/kg in the cynomolgus monkey model of IMQ-induced psoriasis.

**Table 15 Effects of HB0034 on IL-17 and IL-36 levels in skin tissues of cynomolgus monkey model of IMQ-induced psoriasis**

| Group | IL-17 concentration (pg/mL) | IL-36 concentration (pg/mL) |
|---|---|---|
| Group A: normal saline | 17.4±0.84 | 12.49±1.81 |
| Group B: dexamethasone | 8.62±0.59^{∗∗} | 5.27±0.52^{∗∗} |
| Group C: HB0034, 5 mg/kg | 10.25±1.73^{∗∗} | 5.97±1.12^{∗} |
| Group D: HB0034, 15 mg/kg | 8.59±0.85^{∗∗} | 2.97±0.95^{∗∗} |
| Group E: HB0034, 50 mg/kg | 7.34±0.07^{∗∗} | 0.96±0.69^{∗∗} |

| | | |
|---|---|---|
| Note: mean ± SEM, n=6; ^{∗} p<0.05, ^{∗∗} p<0.01 v.s. normal saline. | | |

All documents mentioned in the present application are hereby incorporated by reference in their entirety, just as each document is cited separately as a reference. In addition, it should be understood that various modifications and changes may be made by those skilled in the art after reading the above teachings of the present application and these equivalent forms also fall within the scope defined by the claims appended hereto.

## Claims

1. An antibody, comprising:
(1) a heavy-chain variable region comprising the following three complementary determining regions CDRs:
CDR1 as set forth in SEQ ID NO: 3,
CDR2 as set forth in SEQ ID NO: 4, and
CDR3 as set forth in SEQ ID NO: 5; and
(2) a light-chain variable region comprising the following three complementary determining regions CDRs:
CDR1' as set forth in SEQ ID NO: 6,
CDR2' as set forth in SEQ ID NO: 7, and
CDR3' as set forth in SEQ ID NO: 8.

2. The antibody according to claim 1, wherein said antibody is selected from the group consisting of: animal-derived antibodies, chimeric antibodies, humanized antibodies, or combinations thereof.

3. The antibody according to claim 1, wherein said heavy-chain variable region of said antibody has a sequence as set forth in SEQ ID NO: 1 or 9; and/or
said light-chain variable region of said antibody has a sequence as set forth in SEQ ID NO: 2, 10, 11 or 12.

4. A recombinant protein, comprising:
(i) the antibody according to claim 1; and
(ii) an optional tag sequence assisting expression and/or purification.

5. A CAR construct, wherein an scFv segment of an antigen-binding region of a monoclonal antibody of said CAR construct specifically binds to a binding region of IL-36R,
and, a heavy-chain variable region of said scFv comprises the following three complementary determining regions CDRs:
CDR1 as set forth in SEQ ID NO: 3,
CDR2 as set forth in SEQ ID NO: 4, and
CDR3 as set forth in SEQ ID NO: 5; and
a light-chain variable region of said scFv comprises the following three complementary determining regions CDRs:
CDR1' as set forth in SEQ ID NO: 6,
CDR2' as set forth in SEQ ID NO: 7, and
CDR3' as set forth in SEQ ID NO: 8.

6. A recombinant immune cell, expressing the exogenous CAR construct according to claim 5.

7. An antibody-drug conjugate, comprising:
(a) an antibody moiety, wherein said antibody moiety is the antibody according to claim 1; and
(b) a conjugated moiety conjugated to the antibody moiety, wherein said conjugated moiety is selected from the group consisting of: detectable markers, drugs, toxins, cytokines, radionuclides, enzymes, or combinations thereof.

8. A use of an active ingredient, wherein said active ingredient is selected from the group consisting of: the antibody according to claim 1, the recombinant protein according to claim 4, the CAR construct according to claim 5, the immune cell according to claim 6, the antibody-drug conjugate according to claim 7, or a combination thereof, wherein said active ingredient is used for (a) preparation of a detection reagent or kit; and/or (b) preparation of a drug for prevention and/or treatment of IL-36-related diseases.

9. The use according to claim 8, wherein said IL-36-related diseases comprise psoriasis.

10. A pharmaceutical composition, comprising:
(i) an active ingredient selected from the group consisting of: the antibody according to claim 1, the recombinant protein according to claim 4, the CAR construct according to claim 5, the immune cell according to claim 6, the antibody-drug conjugate according to claim 7, or a combination thereof; and
(ii) a pharmaceutically acceptable carrier.

11. A polynucleotide, wherein said polynucleotide encodes the antibody according to claim 1, the recombinant protein according to claim 4, or the CAR construct according to claim 5.

12. A vector, comprising the polynucleotide according to claim 11.

13. A genetically engineered host cell, wherein said host cell comprises the vector according to claim 12 or a genome integrated with the polynucleotide according to claim 11.

14. A non-diagnostic method for *in vitro* detection of an IL-36R protein in a sample, wherein said method comprises the steps of:
(1) contacting, *in vitro,* the sample with said antibody according to claim 1 or said antibody-drug conjugate according to claim 7;
(2) detecting whether an antigen-antibody complex is formed, wherein the formation of the complex indicates the presence of the IL-36R protein in the sample.

15. A detection plate, wherein said detection plate comprises: a substrate (support plate) and a detection strip, wherein said detection strip comprises said antibody according to claim 1 or said antibody-drug conjugate according to claim 7.

16. A kit, comprising:
(1) a first container, wherein said first container comprises said antibody according to claim 1; and/or
(2) a second container, wherein said second container comprises a secondary antibody targeting said antibody according to claim 1;
or, said kit comprises the detection plate according to claim 15.

17. A preparation method for a recombinant polypeptide, comprising:
(a) culturing said host cell according to claim 13 under conditions suitable for expression; and
(b) isolating a recombinant polypeptide from a culture, wherein said recombinant polypeptide is said antibody according to claim 1 or said recombinant protein according to claim 4.
